(19) **Europäisches Patentamt**
**European Patent Office**
**Office européen des brevets**

(11) **EP 4 399 273 B1**

(12) **EUROPEAN PATENT SPECIFICATION**

(45) Date of publication and mention
of the grant of the patent:
**14.01.2026 Bulletin 2026/03**

(21) Application number: **22772893.8**

(22) Date of filing: **01.09.2022**

(51) International Patent Classification (IPC):
*C12M 1/00* (2006.01)     *C12M 1/26* (2006.01)
*C12M 1/42* (2006.01)

(52) Cooperative Patent Classification (CPC):
**C12M 35/04; C12M 29/04; C12M 33/00**

(86) International application number:
**PCT/EP2022/074275**

(87) International publication number:
**WO 2023/031313 (09.03.2023 Gazette 2023/10)**

(54) **CELL CULTURE SYSTEM COMPRISING COMPARTMENTS AND AN ACOUSTIC ACTUATION DEVICE AND METHODS THEREOF**

ZELLKULTURSYSTEM MIT FÄCHERN UND EINER AKUSTISCHEN BETÄTIGUNGSVORRICHTUNG UND VERFAHREN DAFÜR

SYSTÈME DE CULTURE CELLULAIRE COMPRENANT DES COMPARTIMENTS ET UN DISPOSITIF D'ACTIONNEMENT ACOUSTIQUE ET PROCÉDÉS ASSOCIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.09.2021 EP 21306217**

(43) Date of publication of application:
**17.07.2024 Bulletin 2024/29**

(73) Proprietor: **Kolibri SAS**
**75116 Paris (FR)**

(72) Inventors:
• **DUMY, Gabriel**
**75011 PARIS (FR)**
• **QUILICHINI, Amélie**
**75016 PARIS (FR)**

(74) Representative: **Hoyng Rokh Monegier B.V.**
**Rembrandt Tower, 30th Floor**
**Amstelplein 1**
**1096 HA Amsterdam (NL)**

(56) References cited:
**EP-A1- 1 121 555**     **EP-A1- 3 360 955**
**WO-A1-2019/140019**     **US-A1- 2019 292 510**

**Description**

FIELD OF THE INVENTION

The present invention relates to a cell culture system and methods thereof. Notably, the present invention relates to a cell culture system comprising a culture chamber which comprises a flow compartment and a culture compartment, the cell culture system further comprising an acoustic actuation device. The present invention relates to a method thereof for culturing cells using such cell culture system by maintaining with the acoustic actuation device at least one cell introduced in the culture compartment.

BACKGROUND PRIOR ART

More and more complex biologics are being exploited in modern pharmacopoeia such as antibodies, stem cells, viral vectors, etc. These biologics are not manufactured by chemical synthesis, i.e. through the reaction of several chemical molecules, but by living cells. Therefore, these cells need to be cultured in mass to reach the required quantities. These cells are usually mammalian cell lines, for which the historic means of culture are bioreactors. Alternatively, insect cells have also been used in conjunction with the baculovirus expression vector system. A bioreactor is a device that allow for the culture of a biological objects automatically, in a growth volume. Usually, these devices comprise a culture medium tank where agitation, culture, oxygenation, and pH regulation take place. The biological objects are usually included in the culture medium, where their whole growth cycle takes place.

However these bioreactors are designed for monocellular organisms such as yeasts and bacteria and are poorly adapted for culturing cells of multicellular organisms. The issue is that the cells of multicellular organisms are fragile and are not used to being grown on their own. Traditional mammalian cells usually grow surrounded by their peers, and usually in a well-regulated organism such as humans.

In addition to that, most of the bioproduction capacities that are adapted to these monocellular organisms have evolved from bacterial and yeast bioreactors, which are cells well suited to suspension culture in hostile environments. On the contrary, cells from multicellular organisms and in particular mammalian cells are adherent cells, meaning that they usually grow attached to a substrate. There is thus a technological gap between a demand and the reality of biology in this domain.

Adherent culture techniques have been developed, usually by repeating in parallel a simple validated mean of culture. For instance some techniques use an adherent support and multilayer/vertical stacking of culture surfaces. However these techniques do not allow a sufficient production to meet the biologics demand.

Antibodies being historically the first biologic produced on a massive scale, most of the mammalian cell bioreactor literature reports to them. However, traditional bioreactors are not suitable with all the cells lines that are able to produce biologics of interest and some adaptation have to be made since these ones are not adapted for instance to suspension culture.

A first solution is to design cell lines that are adapted in a culture in suspension in order to increase efficiency. Starting with an adherent cell line, it is generally possible by adjusting the culture and feeding conditions to generate a mutant clone that will be adapted to suspension growth (Berg DT et al, High-level expression of secreted proteins from cells adapted to serum-free suspension culture. Biotechniques. 1993 Jun ; Mcallister, R. et al, Adaptation of Recombinant HEK-293 Cells to Growth in Serum Free Suspension, Animal Cell Technology: Products from Cells, Cells as Products: Proceedings of the 16th ESACT Meeting April 25-29, 1999, Lugano, Switzer*land)*. Then, it is possible to use regular suspension bioreactors (systems derived from bacterial culture, e.g. US10640741B2). Doing so allows for the volumetric growth of a very large number of cells, not being limited by a surface growth. At the end of the culture, it will be necessary to clear the desired biologic from the culture mix.

However the process for obtaining suspension-adapted cell lines is long and costly, especially since a cell line must be issued from a single original clone, which must be selected, tested and stabilized from the original adherent cell lineage (Adaptation of Recombinant HEK-293 Cells to Growth in Serum Free Suspension. Mcallister R., et al., 1999, Animal Cell Technology: Products from Cells, Cells as Products. Springer, Dordrecht; Evolution from adherent to suspension: systems biology of HEK293 cell line development, Malm, M., et al.. Sci Rep 10, 18996, 2020). This process may take multiple years to be completed. Once the stable cell line is designed, there remains issues with the suspension culture of adherent cells, namely because these ones tend to aggregate with each other quite easily, which is something to avoid (Scalable Production of AAV Vectors in Orbitally Shaken HEK293 Cells, Blessing D, et al.. Mol Ther Methods Clin Dev. 2018 Nov 22).

Another solution is to use a carrier allowing volumetric culture, in order to reduce the surface footprint for the culture of very large quantities of adherent cells. Providing a growth support in a volumetric approach is something that can be done by attaching cells to micro carriers. For instance US20140356949A1 discloses a carrier for expansion of induced pluripotent stem cells comprising a substrate with one or more outer surfaces modified with gas plasma treatment and one or more structured indentations on the outer surfaces. Such carrier will then be used in a regular suspension bioreactor.

Another approach could be to attach the cells to a 2.5 D growth support such as a fibrous matrix or a polymer foam, a fractal scaffold that offers a lot of surface in a 3D space (Valkama, A. et al., Optimization of lentiviral vector production for scaleup in fixed-bed bioreactor, Nature, Gene Therapy 25, 39-46, 2018).

However with the use of micro carriers, or with a solid substrate, a volumetric loss is induced by the presence of said substrate. For an equal volume, a carrier based culture will yield lower titers than its suspension equivalent because of this, lessening the improvement. Moreover, the use of a support for adherent cell lines often induces inhomogeneities in gases and nutrients distribution in the case of perfusion bioreactors, and a loss in culture density due to the space taken by the growth support.

The existing solutions described above are oriented towards the use of the already developed bioreactors inherited from the culture of monocellular microorganisms. However, mammalian cells are fundamentally different, and require a very controlled environment. Especially, the shear rates that exist in the use of regular stirred tanks bioreactors is crucial for the good distribution and mixing of gases and nutrients but is very detrimental to these cell lines.

In the past years, reactors using acoustic devices have been developed as they allow holding particles or cells in a reactor. For instance patent application US2016/0369236 A1 particularly discloses a process for continuously collecting cells from a cell culture, the process being performed in a bioreactor including a device for producing multi-dimensional standing waves, which are used to hold a cell culture in place. A nutrient fluid stream is circulated through the bioreactor past the cell culture to collect biological products produced be the cell culture. Patent applications US20180298323 A1 and WO2019140019 A1 disclose acoustic devices comprising an acoustic chamber or reactor and an ultrasonic transducer to create a multi-dimensional acoustic standing wave in the acoustic chamber which help holding particles or cells. Patent application US20170175073 A1 discloses a system comprising a bioreactor, a primary clarification stage with an acoustophoretic separator downstream of and fluidly connected to the bioreactor. In such system, the acoustophoretic separator comprises a flow chamber, an ultrasonic transducer an opposed reflector. These allow producing a multi-dimensional standing wave in the flow chamber.

In the above state of the art, acoustic actuation is generally in direct connection with the volume in which the biologics of interest are cultivated. To be able to culture cells at a high density and for a long period of time, then a renewal of culture medium and gases must take place. However this imposes a condition on the strength of the acoustic field, as any fluid circulation will have to be counteracted by the acoustic field for its benefits to stay. Moreover, acoustic actuation of a liquid layer generates in said liquid a large-scale recirculation commonly named acoustic streaming (Jacob S. Bach and Henrik Bruus, Bulk-driven acoustic streaming at resonance in closed microcavities, Phys. Rev. E 100, 023104, 7 August 2019). This fluid motion can induce additional shear on the fragile cells, and worse, cause them to fall out of their acoustic position if the acoustic field is not strong enough.

There is therefore the need for a system and a method thereof which facilitate the renewal of the culture medium and gases without generating additional shear or any other mechanism which would affect the cultured cells. The invention seeks to overcome the aforementioned drawbacks of the prior art as it aims to prevent any disruption in the environment in which the cells are cultured while providing a scalable technology for the culture of cells and the production of biologics.

SUMMARY OF THE INVENTION

To this effect the invention discloses a cell culture system comprising: a culture chamber comprising: a flow compartment and a culture compartment being separated by a membrane, wherein the membrane is permeable to gas and is acoustically transparent; at least one inlet and one outlet to the flow compartment; at least one inlet and one outlet to the culture compartment; an acoustic actuation device comprising at least a first element being an ultrasonic transducer and an opposed second element being a reflector or a second ultrasonic transducer, wherein the acoustic actuation device is arranged to generate an acoustic field in the culture compartment in order to maintain cells in the culture compartment.

The culture compartment of the cell culture system comprises a first end, a second end opposed to the first end, and a main section between the first end and the second end, wherein the main section of the culture compartment is at least partially situated inside the inner volume of the flow compartment.

Advantageously, the culture compartment of the cell culture system is entirely situated inside the inner volume of the flow compartment.

Advantageously, the distance between the acoustic actuation device and the inner volume of the flow compartment ranges from 500 $\mu$m to 5 mm.

Advantageously, the distance between the first element and the second element of the acoustic actuation device follows the equation (1) being:

$$h_{tot} = n\frac{\lambda}{2} = n\frac{c}{2f} \qquad (1)$$

wherein $h_{tot}$ is the distance between the first and the second element of the acoustic actuation device, $f$ is the acoustic frequency used, c is the propagation speed of the acoustic wave in the liquids between the first and second element of the acoustic actuation device, $\lambda$ is the wavelength of the acoustic wave.

Advantageously, the membrane of the cell culture system is permeable to nutrients.

Advantageously, the walls of the culture chamber of the cell culture system are at least partially constituted by the acoustic actuation device.

Advantageously, the membrane of the cell culture system comprises polyethylene glycol and/or polydimethylsiloxane.

Advantageously, the cell culture system comprises a sampling inlet.

Advantageously, the acoustic actuation device of the cell culture system further comprising a first element comprising a plurality of ultrasonic transducers and an opposed second element comprising a plurality of ultrasonic transducers and/or reflectors, and the plurality of ultrasonic transducers of the first element and the plurality of ultrasonic transducers and/or reflectors of the second element are arranged to generate an acoustic field and to maintain cultured cells in the culture compartment at at least two locations which are separated from each other by a distance ranging from 50 to 500 $\mu$m. The separated cultured cells in the culture compartment may correspond to separated aggregates of cultured cells.

The invention also discloses a set of cell culture systems as defined in any of the previous claims, the systems being parallelized.

The invention also discloses a method for culturing cells using the cell culture system as described previously, the method comprising: flowing a first liquid from the inlet of the flow compartment to the outlet of the flow compartment of the culture chamber and flowing a second liquid from the inlet of the culture compartment to the outlet of the culture compartment of the culture chamber; generating an acoustic field in the culture compartment with the acoustic actuation device; introducing at least one cell in the culture compartment of the culture chamber; and maintaining the at least one cell introduced in the culture compartment.

Advantageously, the excitation frequencies of the ultrasonic transducer of the acoustic actuation device in the method for culturing cells is in the range from 500 kHz to 15 MHz and the excitation amplitude is in the range from 1 to 30 W.

Advantageously, the method for culturing cells further comprises temporarily setting the acoustic actuation device so as to induce cavitation at the location where the at least one cell is maintained.

Advantageously, temporarily setting the acoustic actuation device so as to induce cavitation comprises decreasing the frequency of the acoustic actuation device to a range of 2 to 3 MHz and increasing the amplitude to a range from 200 to 400 kHz.

BRIEF DESCRIPTION OF THE DRAWINGS

The invention will be better understood and its various characteristics and advantages will emerge from the following description of a number of exemplary embodiments and its appended figures in which:

- Figure 1 displays a cross-section of a cell culture system comprising an acoustic actuation device and a cell chamber comprising a flow compartment and a culture compartment, and;
- Figure 2 displays a cross-section of a cell culture system comprising an acoustic actuation device and a cell chamber comprising a flow compartment and a culture compartment, wherein the main section of the culture compartment is situated inside the inner volume of the flow compartment;
- Figure 3 displays a cell culture system comprising an acoustic actuation device and a cell chamber comprising a flow compartment and a culture compartment, wherein the culture compartment is entirely situated inside the inner volume of the flow compartment;
- Figure 4 displays a cell culture system comprising an acoustic actuation device and a cell chamber comprising a flow compartment and multiple culture compartments;
- Figure 5 displays a cross-section of a cell culture system comprising an acoustic actuation device and a cell chamber comprising a flow compartment and a culture compartment;
- Figure 6 displays a cross-section along the A axis of a cell culture system according to figure 1;
- Figure 7 displays a cross-section along the A axis of a cell culture system according to figure 5.

DETAILED DESCRIPTION OF THE INVENTION

In this specification, the invention will be described by way of examples. However, the invention is not restricted to these examples.

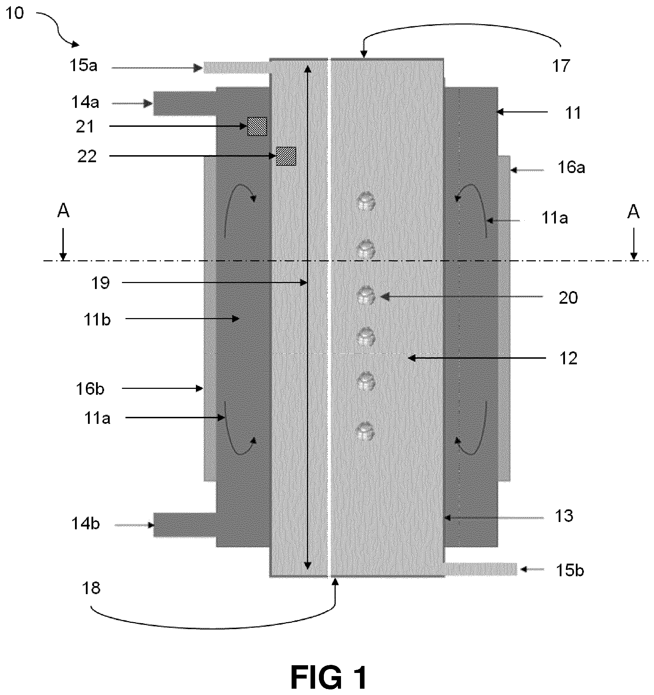

Figure 1 displays a cell culture system 10 comprising a culture chamber comprising a flow compartment 11 and a culture compartment 12, the culture compartment having a first end 17, an opposed second end 18 and a main section 19 between the first end and the second end. A membrane 13 which is gas permeable separates the flow compartment 11 from the culture compartment 12. The culture chamber of figure 1 also comprises an inlet 14a and an outlet 14b to the flow compartment and an inlet 15a and an outlet 15b to the culture compartment. The cell culture system also comprises an acoustic actuation device which comprises a first element 16a which is an ultrasonic transducer and an opposed second element 16b which is a reflector. The first and the second elements of the acoustic actuation device are arranged so as to be able to generate an acoustic field in the culture compartment. Such an acoustic field allows maintaining cells 20 in the culture compartment 12, the cells will adhere progressively to each other and thus provide each other with the necessary growth support. In figure 1, the first and second elements of the acoustic actuation device are fixed on the flow compartment although they may be located elsewhere, for instance on the first end and the second end of the culture compartment. Preferably, the elements of the acoustic actuation device are arranged so as to generate an acoustic field in the center of the culture compartment, e.g. in the center of gravity, so as for the cells 20 to be maintained in the culture compartment 12 without adhering to the membrane 13 and therefore being maintained in suspension.

The cells in the culture compartment, not being bound to a mechanical point, may be manipulated in the volume of the culture compartment by adjusting the phase of the acoustic wave generated by the acoustic actuation device. For instance, adjusting the phase of the acoustic wave allows moving the cells to a sampling outlet, which may correspond to the inlet 15a or the outlet 15b or to another outlet connected to the volume of the culture compartment (not shown).

By flow compartment and culture compartment in the present invention, it is meant a volume physically delimited by walls or, at the separation between the flow compartment and the culture compartment, delimited by a membrane. Although it is preferred that the separation between the flow compartment and the culture compartment is constituted by the membrane 13, such separation may partially comprise walls. Preferably, and this is not limited to the cell culture system of figure 1, these walls are biocompatible, do not release particles in the liquid with which they are in contact, are acoustically transparent, and are manufacturable by injection or moulding. For instance, such walls may be made of materials comprising polypropylene or polystyrene, or being obtained from polymer resin adapted for stereolithography.

The inlet 14a allows introducing a liquid in the flow compartment 11 which will then exit through the outlet 14b. The liquid to introduce in the flow compartment may comprise gases or nutrients and combinations thereof. The inlet 15a allows introducing a liquid in the culture compartment 12 which will then exit through the outlet 15b. The liquid to introduce in the culture compartment may comprise gases or nutrients and combinations thereof. In particular, the inlet 15a of the culture compartment 12 allows introducing one or more cells in the culture compartment. The outlet 15b of the culture compartment 12 allows collecting the one or more cells which were introduced previously and which may have been maintained and cultured in the culture compartment. By "cultured cell", it is meant a cell which has been maintained in the culture compartment 12 and which may have undergone for instance multiplication, differentiation or maturation.

In figure 1, the culture compartment 12 has a first end 17, a second end 18 opposed to the first end and a main section 19 between the first end and the second end. The main section 19 extends from the first end to the second end as indicated by the double arrow of figure 1 and corresponds to the culture compartment except the first and second ends 17, 18 and the inlet and outlet 15a, 15b. The main section 19 of the culture compartment 12 is partially situated inside the inner volume 11b of the flow compartment 11 while the first end 17 and the second end 18 of the culture compartment are not situated inside the inner volume of the flow compartment. Preferably, the exchange surface between the culture compartment and the flow compartment, which corresponds to the membrane separating these compartments, is the largest possible so as to maximize the exchange between the liquids in the flow compartment and in the culture compartment. Although not mandatory, when at least the main section of the culture compartment is partially situated inside the inner volume 11b of the flow compartment 11, the exchange surface between the culture compartment and the flow compartment is increased.

In figure 1, a first group of sensors 21 is situated in the flow compartment 11 and a second group of sensors 22 is situated in the culture compartment 12. These groups of sensors may be placed in different locations in the corresponding compartment. Alternatively, the first and/or second groups of sensors may be situated in the inlet and/or outlet of the flow or culture compartments. Further, the groups of sensors may be connected to one or more controllers which are situated outside the compartments. The connection between the groups of sensors and the controllers may comprise wires or may be wireless. The groups of sensors 21, 22 may be able to monitor pH, temperature, dissolved oxygen, carbon dioxide, pressure, flux rate, shearing rate, etc.

The cell culture system 10 of figure 1 allows introducing a liquid comprising at least one cell 20 in the culture compartment 12 via the inlet 15a and maintaining the at least one cell inside the culture compartment with the first and second elements 16a, 16b of the acoustic actuation device. The at least one cell 20 is provided with gases for its culture, differentiation or maturation with the liquid which is fed through inlet 14a of the flow compartment, the gases passing through the membrane 13. The membrane 13 prevents the turbulences 11a, generated in the flow compartment 11 by the feeding of a liquid through inlet 14a, from being transmitted in the culture compartment 12. Indeed, the turbulences 11a would generate shearing in the environment of the cultured cells, causing mechanical or biological stress to the cells and impacting their multiplication, differentiation or maturation. In the worst case, and depending on the cells to be cultured, shear would cause several cells to die. Notably, the cell culture system of the present invention does not need impellers or blades for mixing the content of the culture compartment.

Therefore a liquid introduced through inlet 14a at a feeding rate which would generate a turbulent flow may still be introduced in the flow compartment, the membrane preventing the turbulences from being transmitted to the culture compartment. Moreover, as the feeding rate is not limited to the turbulences which may be generated, the renewal of the liquid in the flow compartment may be carried out faster and the gas supplied to the culture compartment may be greater and regulated more finely and/or faster. In addition, the liquid introduced into the flow compartment may comprise bubbles without causing harm to the cells. Bubbles may be introduced in the flow compartment for instance within a liquid introduced through the inlet of the flow compartment. Bubbles may increase the gas exchanges between a liquid introduced in the flow compartment and the liquid already present in the flow compartment. Accordingly, the increase of gas exchanges within the flow compartment will also increase gas exchanges between the flow compartment and the culture compartment. Usually, bubbles when conducting acoustic manipulation are a must-avoid because they generate high shear when oscillating under the acoustic actuation. However, according to the cell culture system of the present invention, the membrane prevents any shear from being transmitted to the culture compartment and to the cells.

Furthermore, as the renewal of the liquid in the flow compartment may be carried faster, the control of the temperature, the pH, the dissolved oxygen or carbon dioxide and concentrations of chemicals in the flow compartment and in the culture compartment may be regulated more finely and/or faster. The culture system of the present invention allows renewing the gas content in the liquid in the culture compartment while avoiding changing the flux rate in the culture compartment.

Although not represented in figure 1, in three dimensions the cell culture system of figure 1 and particularly the culture chamber may have a global shape of a right circular cylinder, a square cylinder or any other shapes. The culture chamber may also have a cuboid shape such as a rectangular cuboid, so as to comprise flat and parallel surfaces on which the acoustic actuation device may be fixed.

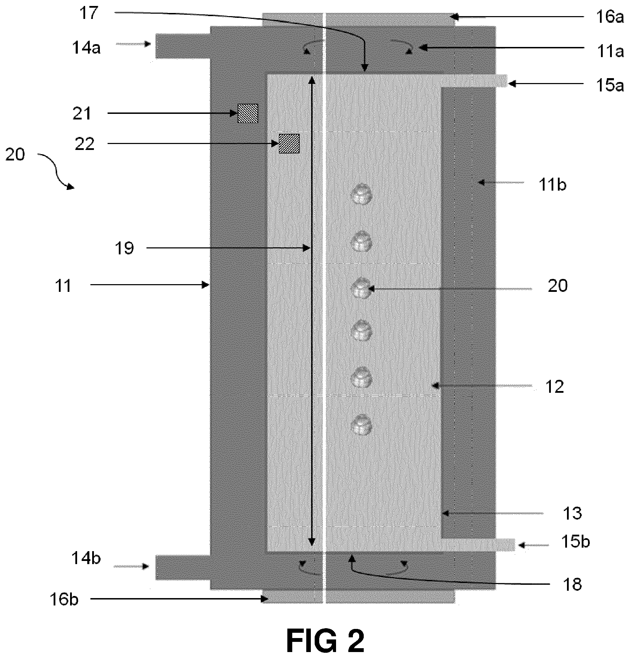

Figure 2 displays a cell culture system 20 comprising a culture chamber comprising a flow compartment 11 and a culture compartment 12, the culture compartment having a first end 17 and an opposed second end 18 and a main section 19 between the first end and the second end of the culture compartment. A membrane 13 which is gas permeable separates the flow compartment 11 from the culture compartment 12. The culture chamber of figure 2 also comprises an inlet 14a and an outlet 14b to the flow compartment and an inlet 15a and an outlet 15b to the culture compartment. The cell culture system also comprises an acoustic actuation device which comprises a first element 16a which is an ultrasonic transducer and an opposed second element 16b which is a reflector. The first and the second elements are arranged so as to be able to generate an acoustic field in the culture compartment. In figure 2, the first end, the second end and the main section of the culture compartment are situated inside the inner volume 11b of the flow compartment 11. The cell culture system also comprises similar groups of sensors 21 and 22 as in figure 1, the groups being situated in the flow compartment 11 and in the culture compartment 12.

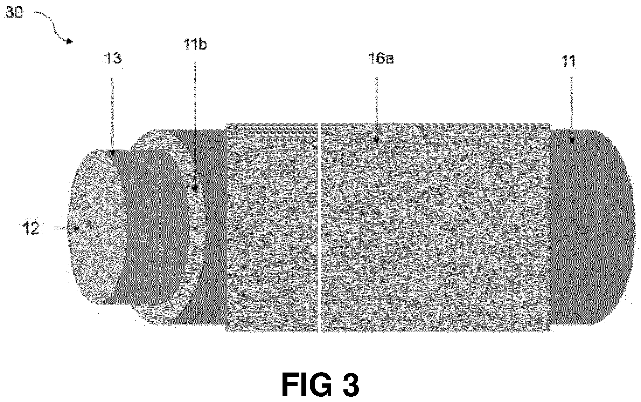

Figure 3 displays a cell culture system 30 comprising an acoustic actuation device comprising a first element 16a and an opposed second element (not visible) and a cell chamber comprising a flow compartment 11 and a culture compartment 12, wherein the culture compartment is entirely situated inside the inner volume 11b of the flow compartment 11. Figure 3 only displays a part of the cell culture system so as to illustrate the culture compartment inside the flow compartment. A membrane 13 separates the volume of the flow compartment and the volume of the culture compartment. In figure 3, the flow compartment has the shape of a right circular cylinder which surrounds the culture compartment which also has the shape of a right circular cylinder, the flow compartment and the culture compartment sharing the same axis. Accordingly, the flow compartment 11 may be provided with a liquid comprising gases so as for the gases to be transmitted via the membrane 13 to the liquid in the culture compartment 12 so as to supply the cells.

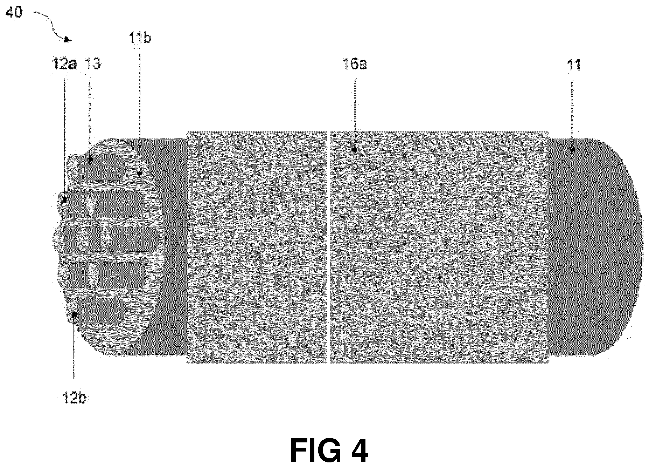

Figure 4 displays a cell culture system 40 comprising an acoustic actuation device comprising a first element 16a and an opposed second element (not visible) and a cell chamber comprising a flow compartment 11 and a culture compartment 12, wherein the culture compartment comprises multiple right circular cylinders having their axis parallel to the axis of the flow compartment. All the right circular cylinders of the culture compartment are entirely situated inside the inner volume 11b of the flow compartment 11. A membrane 13 separates the volume of the flow compartment and the volume of the multiple right circular cylinders of the culture compartment. In figure 4, the flow compartment surrounds the multiple right circular cylinders of the culture compartment. Such embodiment allows growing different type of cells at the same time, while supplying the gas necessary for the cell culture with only the liquid provided in the flow compartment. Each right circular cylinder of the culture compartment may be connected to an inlet and an inlet or they may share the same inlet and outlet.

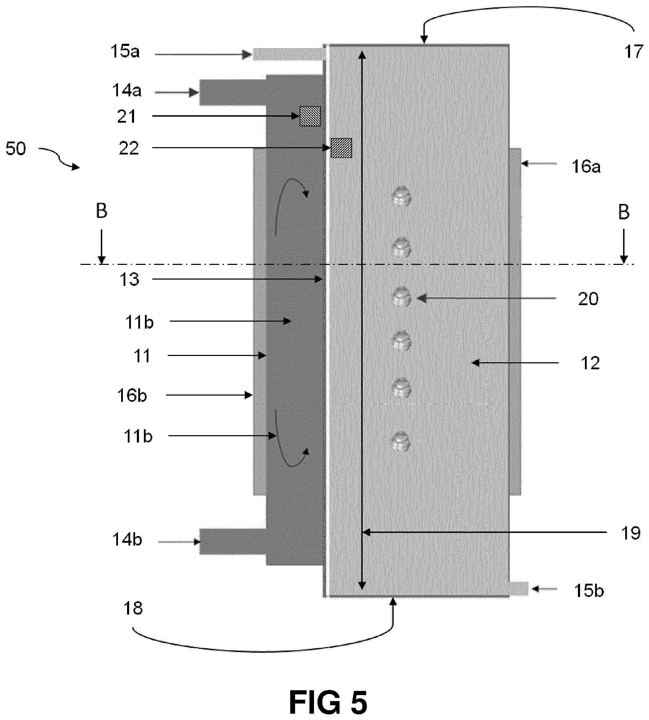

Figure 5 displays a cell culture system 50 comprising a culture chamber comprising a flow compartment 11 and a culture compartment 12, the culture compartment having a first end 17 and an opposed second end 18 and a main section 19 between the first end and the second end of the culture compartment, indicated by a double arrow. A membrane 13 which is gas permeable separates the flow compartment 11 from the culture compartment 12. In other words, the interface between the flow compartment and the culture compartment is at least partially made of a membrane 13. The culture chamber of figure 5 also comprises an inlet 14a and an outlet 14b to the flow compartment and an inlet 15a and an outlet 15b to the culture compartment. The cell culture system also comprises an acoustic actuation device which comprises a first element 16a which is an ultrasonic transducer and an opposed second element 16b which is a reflector. The first and the second elements are arranged so as to be able to generate an acoustic field in the culture compartment. In figure 5, the first end and the second end of the culture compartment do not constitute an interface between them and the flow compartment 11 while the main section of the culture compartment, which extends along the double arrow, faces the flow compartment, or in other words constitute an interface between the flow compartment and the main section. The cell culture system also comprises similar groups of sensors 21 and 22 as in figure 1, the groups of sensors being situated in the flow compartment 11 and in the culture compartment 12 respectively. Unlike the cell culture systems of figures 1 and 2, the main section 19 of the culture compartment is not situated inside the inner volume 11b of the flow compartment 11 but faces the flow compartment side by side. In other words, the flow compartment and the culture compartment are adjoined or side by side at least among a part of the main section of the culture compartment.

The elements of the cell culture system of figure 5 are arranged so as for the actuation device to generate an acoustic field in the center of the culture compartment so as for the cells 20 to be maintained in the culture compartment 12 without adhering to the membrane 13 and therefore being maintained in suspension.

In the cell culture system 50 of figure 5, the main section of the culture compartment, which is represented along arrow 21, is not situated inside the inner volume 11b of the flow compartment 11 but faces the flow compartment. The interface between the main section of the culture compartment and the flow compartment consists in or comprises at least partially a membrane 13.

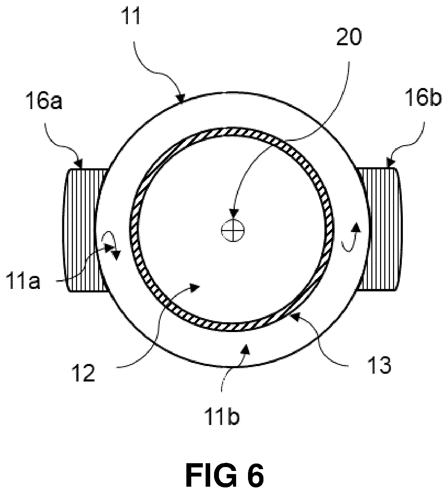

Figure 6 displays a cross-section along the A axis of a cell culture system according to figure 1. Although represented in figure 6 as having a global shape of a right circular cylinder, the cell culture system of the invention is not limited to such right circular cylinder shape and other shapes are possible such as a square cylinder or any other shapes. The cell culture system of figure 6 comprises an acoustic actuation device which comprises a first element 16a which is an ultrasonic transducer and an opposed second element 16b which is a reflector or another ultrasonic transducer. The first and the second elements are arranged so as to be able to generate an acoustic field in the culture compartment 12. In figure 6, the culture compartment 12 is situated inside the inner volume 11b of the flow compartment 11. A membrane 13 prevents the turbulences 11a, generated in the flow compartment 11 by the feeding of a liquid from being transmitted in a culture compartment 12, where the cells 20 are maintained by the acoustic actuation device.

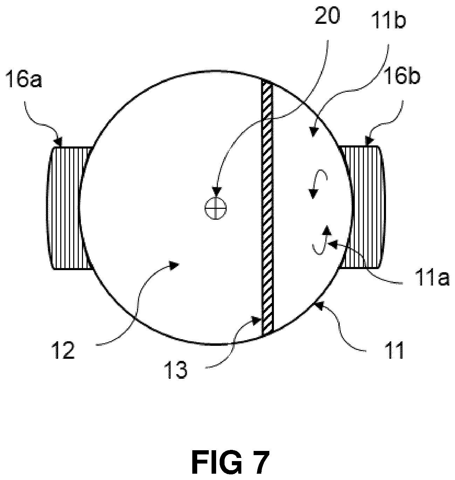

Figure 7 displays a cross-section along the B axis of a cell culture system according to figure 5. Although represented in figure 7 as having a global shape of a right circular cylinder, the cell culture system of the invention is not limited to such right circular cylinder shape and other shapes are possible such as a square cylinder or any other shapes. The cell culture system of figure 7 comprises an acoustic actuation device which comprises a first element 16a which is an ultrasonic transducer and an opposed second element 16b which is a reflector or another ultrasonic transducer. The first and the second elements are arranged so as to be able to generate an acoustic field in the culture compartment 12. In figure 7, the culture compartment is adjoined to the flow compartment, the interface between them consisting in or comprising at least partially a membrane 13. The membrane 13 prevents the turbulences 11a, generated in the flow compartment 11 by the feeding of a liquid from being transmitted in a culture compartment 12, where the cells 20 are maintained by the acoustic actuation device.

The membrane 13 in figures 1 to 7 is acoustically transparent, meaning that its acoustic impedance is the same as the liquid in the culture compartment. In particular embodiments, the membrane is also permeable to nutrients, which allows introducing nutrients in the flow compartment so as they diffuse in the culture compartment through the membrane. The membrane may be flexible and therefore the culture compartment may be a bag suspended in the inner volume of the flow compartment. Preferably, the membrane 13 is made of biocompatible materials. Membrane 13 may comprise polyethylene glycol and/or polydimethylsiloxane (PDMS) or may consist in polyethylene glycol and/or polydimethylsiloxane (PDMS).

In figures 1, 2 and 5, the main section 19 extends from the first end 17 to the opposed second end 18 and is represented by an arrow. In other words, the first end 17 and the opposed second end 18 consist in or comprise at least walls or a part of the membrane 13 separating the culture compartment from the exterior of the cell culture system or the flow compartment, or even from the actuation device in some embodiments. The main section 19 extends from the first end to the opposed second end and represents both the walls, being at least partially composed of the membrane, and the internal volume of the culture compartment. The inlet part 15a and the outlet part 15b of the culture compartment are not part of the main section. In other words, the main section corresponds to the culture compartment with the exception of the ends that flank this culture compartment and the inlet and outlet of the culture compartment.

Preferably, the distance between the first element and the second of the acoustic actuation device is dependent on the acoustic frequency used and follows the equation (1):

$$h_{tot} = n\frac{\lambda}{2} = n\frac{c}{2f} \qquad (1)$$

wherein $h_{tot}$ is the distance between the first and the second element of the acoustic actuation device, $f$ is the acoustic frequency used, c is the propagation speed of the acoustic wave in the liquids between the first and second element of the acoustic actuation device, $\lambda$ is the wavelength of the acoustic wave. When the distance between the first element and the second of the acoustic actuation device follows equation (1) the acoustic waves generated by the ultrasonic transducers and the waves reflected by the reflectors may resonate with an improved precision so as to generate an acoustic field to maintain the cells in the culture compartment with a finer control of the acoustic field. Also, when the distance between the first element and the second of the acoustic actuation device follows equation (1), the acoustic waves generated by the ultrasonic transducers and the waves reflected by the reflectors may have increased amplitude, reducing the need for actuation of the piezoelectric transducers.

Preferably, the thickness of the walls of the flow compartment which are contiguous with the ultrasonic transducer of the acoustic actuation device is dependent on the acoustic frequency used and follows the equation (2):

$$h_{wall} = (2n + 1)\frac{\lambda}{4} = (2n + 1)\frac{c}{4f} \qquad (2)$$

wherein $h_{wall}$ is the thickness of the walls of the flow compartment which are contiguous with the ultrasonic transducer of the acoustic actuation device, $f$ is the acoustic frequency used, c is the propagation speed of the acoustic wave in the walls of the flow compartment, $\lambda$ is the wavelength of the acoustic wave. When the thickness of the walls of the flow compartment follows equation (2), the acoustic waves generated are transmitted through the walls without being impacted, which allows a finer control of the acoustic field generated to maintain the cells in the culture compartment.

Preferably, the cell culture system of the invention comprises a total volume of a flow compartment and a culture compartment of 1 milliliter to 1 000 liters, even more preferably from 200 liters to 1 000 liters. A culture system of a total volume of 1 mL to 250 mL is particularly adapted for optimizing the process, for parametric exploration or paralleling experiments. A culture system of a total volume of 1 L to 80 L is particularly adapted for preclinical production, animal testing or small therapies. A culture system of a total volume over 150 L is particularly adapted for industrial production and clinical trials gene therapy.

Preferably, the ultrasonic transducers of the present invention comprise piezoelectric ceramics, such as PZT ceramics although the invention is not limited to PZT ceramics. According to the system of the invention, excitation frequencies in the range from 100 kHz to 20 MHz and preferably from 500 kHz to 15 MHz are applied by the ultrasonic transducer. Further, the excitation amplitude is in the range from 1 to 1000 W. In particular, the excitation amplitude is at minimum of 1,5 W per liter. Therefore in the case of a cell culture system of 1 liter, the excitation amplitude is from 1,5 to 30W, in the case of a cell culture of 10 L, the excitation amplitude is from 15 to 200W and in the case of a cell culture of 100 L, the excitation amplitude is from 150 to 1000 W. The ultrasonic transducers of the acoustic actuation device may also be flexible so that they may adapt to the shape of the flow compartment of the cell culture system.

For instance, the typical size of an ultrasonic transducer of the first or second element of the acoustic actuation device is of 70 x 70 millimeter, the thickness depending on the frequency, being of 0.5 mm for a frequency of 4 MHz, 1 mm for a frequency of 2 MHz and 2 mm for a frequency of 1 MHz.

The reflectors of the second element of the acoustic actuation device have a significant impedance break with the culture medium in the culture compartment. Materials with a high acoustic impedance are therefore preferred, such as steel with a density of 8 $g/cm^3$, speed of sound of 5521 m/s and an acoustic impedance of 44,2 MPa·$s/m^3$, for instance stainless steel 316 or steel which are used in the biomedical industry, or such as aluminum with a density of 2,7 $g/cm^3$, speed of sound of 6108 m/s and an acoustic impedance of 16,5 MPa·$s/m^3$.

Preferably, the thickness of the membrane ranges from 50 $\mu$m to 2 mm. Preferably, the size of the pores of the membrane ranges from 0,2 to 4 $\mu$m, which prevents the cultured cells from exiting the culture compartment.

Preferably, the sensors are configured to monitor multiple parameters and to warn if a threshold of a parameter has been exceeded. For instance, the pH should range from 6 to 8, the temperature from 30 to 40°C, the dissolved oxygen from 0 to 20 mg/L, the carbon dioxide from 0 to 100 mg/L, the pressure from 0 to 1 bar and the flux rate from 0 to 20 L/min.

In a particular embodiment, which may be combined with the other embodiments disclosed herein, the first element and/or the second element of the acoustic actuation device may be integrated in the walls of the culture chamber, e.g. in the walls of the flow compartment or of the culture compartment. Alternatively, the acoustic actuation device may constitute a part of the walls of the flow compartment or of the culture compartment.

The cells to be cultured in the cell culture system of the invention may be bacteria, e.g. cyanobacteria, plant cells, e.g. algae, animal cells, e.g. insect cells or mammalian cells (human or non-human) such as mesenchymal stem cells, embryonic stem cells, induced pluripotent stem cells, hematopoietic stem cells, T-lymphocytes (cart-T), Human Embryonic Kidney cells (HEK) or others. In the case of human embryonic stem cells, these are obtained only without destruction of the embryo from which they are derived and are not suitable for inducing the developmental process of a human being.

The system of the invention allows enabling the cells to grow in volume, to save time for their multiplication and to optimize it, while avoiding the need for growth support consumables.

The system of the invention may be associated to other similar systems of the invention as a set of systems, the systems being parallelized in order to increase the global capacity of the overall set of systems. In a preferred embodiment these systems have a rectangular cuboid shape so that they may be stacked, allowing them, inter alia, to occupy as minimal space as possible. In a set of systems, the groups of sensors may be connected to one or more controller to supervise the overall set of systems or a particular system among the systems of the set of systems.

Example

In a particular example, the cell culture system comprises an acoustic actuation device comprising a first element comprising 3 ultrasonic transducers and an opposed second element comprising 3 reflectors, each reflector being opposed to an ultrasonic transducer so as to be able to generate an acoustic field in the culture compartment. The ultrasonic transducers and the reflectors size is 70 x 70 mm and are arranged one after the other to cover a length of 210 mm. The thickness of the walls of the flow compartment follows the equation (3):

$$h = (2n + 1)\frac{\lambda}{4} = (2n + 1)\frac{c}{4f} \qquad (3)$$

wherein $h$ is the thickness of the thickness of the walls of the flow compartment, $f$ is the acoustic frequency used, $c$ is the propagation speed of the acoustic wave in the walls of the flow compartment, $\lambda$ is the wavelength of the acoustic wave in the material composing the walls.

In this example, the membrane is made of polydimethylsiloxane (PDMS) which prevents the transmission of liquid turbulence from the flow compartment to the culture compartment, but other materials may be used for the membrane.

**Claims**

1. A cell culture system (10) comprising:

    a culture chamber comprising:

        - a flow compartment (11) and a culture compartment (12) being separated by a membrane (13), wherein the membrane is permeable to gas and is acoustically transparent;
        - at least one inlet (14a) and one outlet (14b) to the flow compartment;
        - at least one inlet (15a) and one outlet (15b) to the culture compartment;

    an acoustic actuation device comprising at least a first element (16a) being an ultrasonic transducer and an opposed second element (16b) being a reflector or a second ultrasonic transducer, wherein the acoustic actuation device is arranged to generate an acoustic field in the culture compartment in order to maintain cells in the culture compartment, and wherein the culture compartment (12) comprises a first end (17), a second end (18) opposed to the first end, and a main section (19) extending from the first end to the second end, wherein the main section of the culture compartment is at least partially situated inside the inner volume (11b) of the flow compartment (11).

2. The cell culture system (20) of claim 1, wherein the culture compartment (12) is entirely situated inside the inner volume (11b) of the flow compartment (11b).

3. The cell culture system (10) of any of the previous claims, wherein the distance between the acoustic actuation device and the inner volume of the flow compartment ranges from 500 $\mu$m to 5 mm.

4. The cell culture system (10) of any of the previous claims, wherein the distance between the first element (16a) and the second element (16b) of the acoustic actuation device follows the equation (1) being:

$$h_{tot} = n\frac{\lambda}{2} = n\frac{c}{2f} \qquad\qquad (1)$$

wherein $h_{tot}$ is the distance between the first and the second element of the acoustic actuation device, $f$ is the acoustic frequency used, $c$ is the propagation speed of the acoustic wave in the liquids between the first and second element of the acoustic actuation device, $\lambda$ is the wavelength of the acoustic wave.

5. The cell culture system (10) of any of the previous claims, wherein the membrane (13) is permeable to nutrients.

6. The cell culture system (10) of any of the previous claims, wherein the walls of the culture chamber are at least partially constituted by the acoustic actuation device.

7. The cell culture system (10) of any of the previous claims, wherein the membrane (13) comprises polyethylene glycol and/or polydimethylsiloxane.

8. The cell culture system (10) of any of the previous claims, wherein the system comprises a sampling inlet.

9. The cell culture system (10) of any of the previous claims, the acoustic actuation device further comprising a first element comprising a plurality of ultrasonic transducers (16a, 16c, 16e, 16g) and an opposed second element comprising a plurality of ultrasonic transducers and/or reflectors (16b, 16d, 16f, 16h), and
wherein the plurality of ultrasonic transducers of the first element and the plurality of ultrasonic transducers and/or reflectors of the second element are arranged to generate an acoustic field and to maintain cultured cells (20) in the culture compartment (12) at at least two locations which are separated from each other by a distance ranging from 50 to 500 $\mu$m.

10. A set of cell culture systems as defined in any of the previous claims, the systems being parallelized.

11. A method for culturing cells using the cell culture system (10) of any of the previous claims, the method comprising:

- flowing a first liquid from the inlet (14a) of the flow compartment (11) to the outlet (14b) of the flow compartment of the culture chamber and flowing a second liquid from the inlet (15a) of the culture compartment (12) to the outlet (15b) of the culture compartment of the culture chamber;
- generating an acoustic field in the culture compartment with the acoustic actuation device;
- introducing at least one cell in the culture compartment of the culture chamber; and
- maintaining the at least one cell introduced in the culture compartment.

12. The method of claim 11, wherein the excitation frequencies of the ultrasonic transducer of the acoustic actuation device is in the range from 500 kHz to 15 MHz and the excitation amplitude is in the range from 1 to 30 W.

13. The method of one of the previous claims, wherein the method further comprises temporarily setting the acoustic actuation device so as to induce cavitation at the location where the at least one cell is maintained.

14. The method of claim 13, wherein temporarily setting the acoustic actuation device so as to induce cavitation comprises decreasing the frequency of the acoustic actuation device to a range of 2 to 3 MHz and increasing the amplitude to a range from 200 to 400 kHz.

**Patentansprüche**

1. Zellkultursystem (10), umfassend:

eine Kulturkammer, umfassend:

- ein Strömungsfach (11) und ein Kulturfach (12), die durch eine Membran (13) voneinander getrennt sind, wobei die Membran gasdurchlässig und akustisch transparent ist;
- mindestens einen Einlass (14a) und einen Auslass (14b) zu dem Strömungsfach;
- mindestens einen Einlass (15a) und einen Auslass (15b) zu dem Kulturfach;

eine akustische Betätigungsvorrichtung, die mindestens ein erstes Element (16a), das ein Ultraschallwandler ist, und ein gegenüberliegendes zweites Element (16b), das ein Reflektor oder ein zweiter Ultraschallwandler ist, umfasst,

wobei die akustische Betätigungsvorrichtung dazu eingerichtet ist, ein akustisches Feld in dem Kulturfach zu erzeugen, um Zellen in dem Kulturfach zu halten, und

wobei das Kulturfach (12) ein erstes Ende (17), ein dem ersten Ende gegenüberliegendes zweites Ende (18) und einen sich vom ersten Ende zum zweiten Ende erstreckenden Hauptabschnitt (19) umfasst, wobei sich der Hauptabschnitt des Kulturfachs zumindest teilweise innerhalb des Innenvolumens (11b) des Strömungsfachs (11) befindet.

2. Zellkultursystem (20) nach Anspruch 1, wobei sich das Kulturfach (12) vollständig innerhalb des Innenvolumens (11b) des Strömungsfachs (11b) befindet.

3. Zellkultursystem (10) nach einem der vorhergehenden Ansprüche, wobei der Abstand zwischen der akustischen Betätigungsvorrichtung und dem Innenvolumen des Strömungsfachs in einem Bereich von 500 μm bis 5 mm liegt.

4. Zellkultursystem (10) nach einem der vorhergehenden Ansprüche, wobei der Abstand zwischen dem ersten Element (16a) und dem zweiten Element (16b) der akustischen Betätigungsvorrichtung der Gleichung (1) folgt:

$$h_{tot} = n\frac{\lambda}{2} = n\frac{c}{2f} \qquad\qquad (1)$$

wobei $h_{tot}$ der Abstand zwischen dem ersten und dem zweiten Element der akustischen Betätigungsvorrichtung ist, $f$ die verwendete akustische Frequenz ist, c die Ausbreitungsgeschwindigkeit der akustischen Welle in den Flüssigkeiten zwischen dem ersten und dem zweiten Element der akustischen Betätigungsvorrichtung ist, $\lambda$ die Wellenlänge der akustischen Welle ist.

5. Zellkultursystem (10) nach einem der vorhergehenden Ansprüche, wobei die Membran (13) für Nährstoffe durchlässig ist.

6. Zellkultursystem (10) nach einem der vorhergehenden Ansprüche, wobei die Wände der Kulturkammer zumindest teilweise durch die akustische Betätigungsvorrichtung gebildet sind.

7. Zellkultursystem (10) nach einem der vorhergehenden Ansprüche, wobei die Membran (13) Polyethylenglykol und/oder Polydimethylsiloxan umfasst.

8. Zellkultursystem (10) nach einem der vorhergehenden Ansprüche, wobei das System einen Probenahmeeinlass umfasst.

9. Zellkultursystem (10) nach einem der vorhergehenden Ansprüche, wobei die akustische Betätigungsvorrichtung ferner ein erstes Element, das eine Mehrzahl von Ultraschallwandlern (16a, 16c, 16e, 16g) umfasst, und ein gegenüberliegendes zweites Element umfasst, das eine Mehrzahl von Ultraschallwandlern und/oder Reflektoren (16b, 16d, 16f, 16h) umfasst, und

wobei die Mehrzahl von Ultraschallwandlern des ersten Elements und die Mehrzahl von Ultraschallwandlern und/oder Reflektoren des zweiten Elements dazu eingerichtet sind, ein akustisches Feld zu erzeugen und kultivierte Zellen (20) in dem Kulturfach (12) an mindestens zwei Stellen zu halten, die durch einen Abstand im Bereich von 50 bis 500 μm voneinander getrennt sind.

10. Satz von Zellkultursystemen nach einem der vorhergehenden Ansprüche, wobei die Systeme parallelisiert sind.

11. Verfahren zum Kultivieren von Zellen unter Verwendung des Zellkultursystems (10) nach einem der vorhergehenden Ansprüche, wobei das Verfahren umfasst:

- Strömenlassen einer ersten Flüssigkeit von dem Einlass (14a) des Strömungsfachs (11) zu dem Auslass (14b) des Strömungsfachs der Kulturkammer und Strömenlassen einer zweiten Flüssigkeit von dem Einlass (15a) des Kulturfachs (12) zu dem Auslass (15b) des Kulturfachs der Kulturkammer;

- Erzeugen eines akustischen Feldes in dem Kulturfach mit der akustischen Betätigungsvorrichtung;
- Einführen mindestens einer Zelle in das Kulturfach der Kulturkammer; und
- Halten der mindestens einen in das Kulturfach eingeführten Zelle.

12. Verfahren nach Anspruch 11, wobei die Anregungsfrequenzen des Ultraschallwandlers der akustischen Betätigungsvorrichtung im Bereich von 500 kHz bis 15 MHz liegen und die Anregungsamplitude im Bereich von 1 bis 30 W liegt.

13. Verfahren nach einem der vorhergehenden Ansprüche, wobei das Verfahren ferner das vorübergehende Einstellen der akustischen Betätigungsvorrichtung umfasst, um Kavitation an der Stelle zu induzieren, an der die mindestens eine Zelle gehalten wird.

14. Verfahren nach Anspruch 13, wobei das vorübergehende Einstellen der akustischen Betätigungsvorrichtung, um Kavitation zu induzieren, das Senken der Frequenz der akustischen Betätigungsvorrichtung auf einen Bereich von 2 bis 3 MHz und das Erhöhen der Amplitude auf einen Bereich von 200 bis 400 kHz umfasst.

## Revendications

1. Système de culture cellulaire (10) comprenant :

    une chambre de culture comprenant :

   - un compartiment d'écoulement (11) et un compartiment de culture (12) séparés par une membrane (13), où la membrane est perméable au gaz et est acoustiquement transparente ;
   - au moins une entrée (14a) et une sortie (14b) vers le compartiment d'écoulement ;
   - au moins une entrée (15a) et une sortie (15b) vers le compartiment de culture ;

    un dispositif d'actionnement acoustique comprenant au moins un premier élément (16a) qui est un transducteur ultrasonique et un deuxième élément opposé (16b) qui est un réflecteur ou un deuxième transducteur ultrasonique,
    où le dispositif d'actionnement acoustique est agencé pour générer un champ acoustique dans le compartiment de culture afin de maintenir les cellules dans le compartiment de culture, et
    où le compartiment de culture (12) comprend une première extrémité (17), une deuxième extrémité (18) opposée à la première extrémité, et une section principale (19) s'étendant de la première extrémité à la deuxième extrémité, où la section principale du compartiment de culture est au moins partiellement située à l'intérieur du volume interne (11b) du compartiment d'écoulement (11).

2. Système de culture cellulaire (20) selon la revendication 1, où le compartiment de culture (12) est entièrement situé à l'intérieur du volume interne (11b) du compartiment d'écoulement (11b).

3. Système de culture cellulaire (10) selon l'une quelconque des revendications précédentes, où la distance entre le dispositif d'actionnement acoustique et le volume interne du compartiment d'écoulement est de 500 $\mu$m à 5 mm.

4. Système de culture cellulaire (10) selon l'une quelconque des revendications précédentes, où la distance entre le premier élément (16a) et le deuxième élément (16b) du dispositif d'actionnement acoustique suit l'équation (1) suivante :

$$h_{tot} = n\frac{\lambda}{2} = n\frac{c}{2f} \qquad\qquad (1)$$

où $h_{tot}$ est la distance entre le premier et le deuxième élément du dispositif d'actionnement acoustique, $f$ est la fréquence acoustique utilisée, c est la vitesse de propagation de l'onde acoustique dans les liquides entre le premier et le deuxième élément du dispositif d'actionnement acoustique, $\lambda$ est la longueur d'onde de l'onde acoustique.

5. Système de culture cellulaire (10) selon l'une quelconque des revendications précédentes, où la membrane (13) est perméable aux nutriments.

6. Système de culture cellulaire (10) selon l'une quelconque des revendications précédentes, où les parois de la chambre de culture sont au moins partiellement constituées par le dispositif d'actionnement acoustique.

7. Système de culture cellulaire (10) selon l'une quelconque des revendications précédentes, où la membrane (13) comprend du polyéthylène glycol et/ou du polydiméthylsiloxane.

8. Système de culture cellulaire (10) selon l'une quelconque des revendications précédentes, où le système comprend une entrée d'échantillonnage.

9. Système de culture cellulaire (10) selon l'une quelconque des revendications précédentes, le dispositif d'actionnement acoustique comprenant en outre un premier élément comprenant une pluralité de transducteurs ultrasoniques (16a, 16c, 16e, 16g) et un deuxième élément opposé comprenant une pluralité de transducteurs ultrasoniques et/ou de réflecteurs (16b, 16d, 16f, 16h), et
où la pluralité de transducteurs ultrasoniques du premier élément et la pluralité de transducteurs ultrasoniques et/ou de réflecteurs du deuxième élément sont agencés pour générer un champ acoustique et pour maintenir les cellules cultivées (20) dans le compartiment de culture (12) à au moins deux emplacements qui sont séparés l'un de l'autre par une distance allant de 50 à 500 $\mu$m.

10. Ensemble de systèmes de culture cellulaire tel que défini dans l'une quelconque des revendications précédentes, les systèmes étant parallélisés.

11. Procédé de culture de cellules utilisant le système de culture cellulaire (10) selon l'une quelconque des revendications précédentes, le procédé comprenant :

- la mise en écoulement d'un premier liquide depuis l'entrée (14a) du compartiment d'écoulement (11) vers la sortie (14b) du compartiment d'écoulement de la chambre de culture et la mise en écoulement d'un deuxième liquide depuis l'entrée (15a) du compartiment de culture (12) vers la sortie (15b) du compartiment de culture de la chambre de culture ;
- la génération d'un champ acoustique dans le compartiment de culture avec le dispositif d'actionnement acoustique ;
- l'introduction d'au moins une cellule dans le compartiment de culture de la chambre de culture ; et
- le maintien de l'au moins une cellule introduite dans le compartiment de culture.

12. Procédé selon la revendication 11, où les fréquences d'excitation du transducteur ultrasonique du dispositif d'actionnement acoustique sont dans la plage de 500 kHz à 15 MHz et l'amplitude d'excitation est dans la plage de 1 à 30 W.

13. Procédé selon l'une quelconque des revendications précédentes, où le procédé comprend en outre le réglage temporaire du dispositif d'actionnement acoustique de manière à induire une cavitation à l'emplacement où l'au moins une cellule est maintenue.

14. Procédé selon la revendication 13, où le réglage temporaire du dispositif d'actionnement acoustique de manière à induire une cavitation comprend la diminution de la fréquence du dispositif d'actionnement acoustique dans une plage de 2 à 3 MHz et l'augmentation de l'amplitude dans une plage de 200 à 400 kHz.

**FIG 1**

**FIG 2**

30

13 11b 16a 11

12

FIG 3

**FIG 4**

**FIG 5**

**FIG 6**

**FIG 7**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 10640741 B2
- US 20140356949 A1
- US 20160369236 A1
- US 20180298323 A1
- WO 2019140019 A1
- US 20170175073 A1

### Non-patent literature cited in the description

- **BERG DT et al.** High-level expression of secreted proteins from cells adapted to serum-free suspension culture. *Biotechniques*, June 1993
- **MCALLISTER, R. et al.** Adaptation of Recombinant HEK-293 Cells to Growth in Serum Free Suspension. *Animal Cell Technology: Products from Cells, Cells as Products: Proceedings of the 16th ESACT Meeting April 25-29*, 1999
- Animal Cell Technology: Products from Cells. **MCALLISTER R. et al.** Cells as Products. Springer, 1999
- **MALM, M. et al.** *Sci Rep*, 2020, vol. 10, 18996
- **BLESSING D et al.** Scalable Production of AAV Vectors in Orbitally Shaken HEK293 Cells. *Mol Ther Methods Clin Dev.*, 22 November 2018
- **VALKAMA, A. et al.** , Optimization of lentiviral vector production for scaleup in fixed-bed bioreactor. *Nature, Gene Therapy*, 2018, vol. 25, 39-46
- **JACOB S**. Bach and Henrik Bruus, Bulk-driven acoustic streaming at resonance in closed microcavities. *Phys. Rev. E*, 07 August 2019, vol. 100, 023104